# EUROPÄISCHE PATENTSCHRIFT

(11) **EP 1 418 225 B1**
(45) Veröffentlichungstag und Bekanntmachung des Hinweises auf die Patenterteilung: **29.06.2005**
(21) Anmeldenummer: 03025253.0
(22) Anmeldetag: 06.11.2003
(51) Int. Cl.: C11D 17/00, A61L 9/04, A61L 9/05

(54) **Toilettenreinigungs- und Beduftungsmittel**
Toilet cleaning and odorising agent
Agent nettoyant et odorisant pour les toilettes

(30) Priorität: 08.11.2002 DE 10252542
(43) Veröffentlichungstag der Anmeldung: 12.05.2004
(73) Patentinhaber: Buck-Chemie GmbH ., 71083 Herrenberg (DE)
(72) Erfinder: Dettinger, Johannes, Dr., 72160 Horb (DE); Jaeschke, Edgar, 70794 Filderstadt-Bonladen (DE)
(74) Vertreter: Mammel, Ulrike, Dr.

(56) Entgegenhaltungen:
- EP-A- 0 864 637
- US-A- 4 460 490

## Beschreibung

Die vorliegende Erfindung betrifft ein Reinigungs- und Beduftungsmittel für den Sanitärbereich, insbesondere für Toiletten sowie Verfahren zur Herstellung eines solchen Mittels.

Im Stand der Technik sind verschiedene Toilettenreinigungs- und Beduftungsmittel bekannt.

Ein Großteil der bekannten Mittel sind so genannte "Rimblocks", die in einem Behältnis, insbesondere einem Körbchen oder käfigartigem Behälter, am Rand ("Rim") der Toilette befestigt werden. Das in dem Behältnis befindliche Mittel wird bei jedem Spülvorgang von dem Spülwasser überströmt. Hierdurch wird bei jeder Spülung ein geringer Anteil des Rimblocks unter Freisetzung von Tensiden, Duftstoffen etc. aufgelöst, wodurch dann die gewünschte Reinigung des Toilettenbeckens und Toilettensumpfs und die gewünschte Beduftung erzielt wird.

Die bekannten Rimblocks verbrauchen sich ausschließlich durch den Spülstrom des Wassers, und die Duftstofffreigabe ist von der Spülung der Toilette abhängig. Die erzielte Lebensdauer und der Gehalt an Duftstoffen für die Raumbeduftung sind dabei regelmäßig für den Gebrauch einer drei- bis vierköpfigen Familie ausgelegt, so dass eine ausreichende Raumbeduftung dann nicht mehr erfolgen kann, wenn die Toilette von weniger Personen oder nicht dauerhaft benutzt wird. Die Raumbeduftung wird bei den Rimblocks im wesentlichen dadurch erzielt, dass die Duftstoffe durch das Spülwasser herausgespült werden, in den Toilettensumpf gelangen und ein Teil der Duftstoffe in dem Toilettensumpf verdampft. Werden die bekannten Rimblocks somit in einer weniger frequentierten Toilette eingesetzt, so wird zwar nach der Toilettenbenutzung die gewünschte Reinigung erzielt, allerdings wird infolge zu geringer Spülzahlen nicht die gewünschte anhaltende Raumbeduftung gewährleistet.

Durch die in den bekannten Rimblocks enthaltenen Netzmittel, zum Beispiel in Form von anionischen Tensiden, werden die Duftstoffe zudem mehr oder weniger eingeschlossen, so dass sie nicht im ausreichenden Maße verdampfen können.

Um eine intensive Raumbeduftung zu gewährleisten, müssten somit große Mengen an Duftstoffen eingesetzt werden, was im Hinblick auf ökonomische Überlegungen nachteilig ist. Gleichzeitig würde ein Großteil der teuren Duftstoffe durch den Spülstrom zusammen mit den weiteren Bestandteilen, an denen sie haften, wirkungslos in die Kanalisation gespült.

Um das bestehende Problem, nämlich ein Toilettenreinigungsmittel bereitzustellen, das bei nur geringer Duftstoffdosierung eine ausreichende und permanente Raumbeduftung auch dann ermöglicht, wenn keine Toilettenspülung erfolgt, wurde in der DE 197 10 635 A1 bereits vorgeschlagen, Gelbildner und Lösungsmittel enthaltende Toilettenreinigungsmittel mit Duftstoffen bereitzustellen.

Mit diesen Gelbildner umfassenden Mitteln wird die gewünschte, von der Spülzahl unabhängige Beduftung erreicht. Auch zeigen diese Mittel, die hochwirksame flüssige Schäumer ("Superschäumer") umfassen, eine außerordentlich gute Schaumbildung. Da diese Gelbildner umfassenden Mittel jedoch zur Bildung der Gelstruktur notwendigerweise einen hohen Anteil an Lösungsmitteln umfassen, werden die Wirkstoffe gegenüber den bekannten Rimblocks nur in verdünnter Form bereitgestellt, so dass die erforderliche Reinigungswirkung nicht erzielt werden kann.

Die bei den Gelen durch Einsatz der flüssigen Superschäumer erreichte hervorragende Schaumbildung kann wiederum bei "Rimblocks" nicht erzielt werden, da das zu extrudierende Gemisch zur Herstellung der Rimblocks durch Einsatz der flüssigen Superschäumer klebrig würde, so dass keine ordnungsgemäße Extrusion erreicht werden kann. Jedoch ist durch den Einsatz von üblichen festen Tensiden eine sehr gute Reinigungswirkung erzielbar.

Im Stand der Technik sind weiterhin gelförmige Reinigungs- und Beduftungsmittel bekannt, die jedoch nicht zur Anwendung in einem Toilettenkörbchen, sondern zum direkten Aufbringen auf der Toilettenschüssel vorgesehen sind, wie in der WO 99/66017 und der DE 100 48 887 A1 beschrieben.

Die Aufgabe der vorliegenden Erfindung besteht darin, ein Toilettenreinigungs- und Beduftungsmittel bereitzustellen, das sowohl die gewünschte Reinigungswirkung erzielt, als auch bei geringer Duftstoffdosierung eine ausreichende und permanente Raumbeduftung selbst dann ermöglicht, wenn keine Toilettenspülung erfolgt.

Diese Aufgabe wird durch die Merkmale des Anspruchs 1 gelöst.

Das erfindungsgemäße Mittel umfasst wenigstens zwei Phasen, nämlich eine erste Gelphase, die Gelbildner, Duftstoffe und Lösungsmittel umfasst und eine zweite Phase, die ein nicht gelförmiger Reinigungsmittelformkörper ist, der Tenside enthält.

Die erste Phase, nämlich die Gelphase des Mittels, zeichnet sich durch ein definiertes Abdampfprofil aus; die Freisetzung der Duftstoffe aus der Gelphase erfolgt somit unabhängig von der Anzahl der Spülungen der Toilette. Mit der Gelphase wird somit die gewünschte von der Spülzahl unabhängige Beduftung erreicht. Zusätzlich verbraucht sich die erste Phase auch beim Überspülen mit Spülwasser; das Überspülen ist jedoch nicht erforderlich, um die gewünschte Raumbeduftung bereitzustellen.

Die zweite Phase dient dazu, die gewünschte Reinigungswirkung zu erzielen. Hierzu umfasst die zweite Phase Tenside, wahlweise auch Lösungsretardierer, anorganische Salze, mit denen die Konsistenz und das Abspülverhalten der zweiten Phase beeinflusst werden kann, Farbstoffe, Desinfektionsmittel, Bleichmittel, Aktivatoren, Enzyme, Komplexierungsmittel, Duftstoffe, Extrusionshilfsmittel etc. Im Allgemeinen wird diese zweite Phase durch Mischen der Bestandteile und anschließende Extrusion und/oder Verpressen hergestellt.

Nachfolgend werden die Bestandteile der ersten und der zweiten Phase näher beschrieben:

### Erste Phase (Gelphase):

Wesentliche Bestandteile der Gelphase sind Gelbildner, die für die Gelbildung erforderlichen Lösungsmittel und die für die Beduftung erforderlichen Duftstoffe.

Als Gelbildner können beispielsweise Metallseifen der höheren Fettsäuren, insbesondere die entsprechenden Alkalisalze wie Natriumstearat oder Natriumoleat, eingesetzt werden. Ebenfalls ist möglich, als Gelbildner nichtionische Tenside wie beispielsweise Polyalkoxyalkane, zum Beispiel ein Gemisch aus Alkyl-(C20,C22)-ethoxylat mit 35 EO, Alkyl-(C22)-ethoxylat mit 35 EO oder Alkyl-(C16,C18)-ethoxylat mit 30 EO, natürliche Polymere ("gums") wie Gummi arabicum, Guar-Gum, Agar Agar, Pektine, Gelatine, Stärke, Sorbitol, chemisch modifizierte oder derivatisierte natürliche "gums" wie Carboxymethylcellulose, Methylcellulose, Hydroxypropylmethylcellulose, Xanthan, Sorbitolderivate wie Dibenzylidensorbitol und deren Derivate, Stärkederivate wie Carboxymethylstärke, Hydroxyethylstärke, mikrobiell fermentierte "gums" wie Dextran, Polysaccharid B-1459 und Gelbildner wie Methoxypectin, Propylenglycolalginat, Triethanolaminalginat, Carboxymethyl-Guar-Gum etc. einzusetzen.

Der Anteil des Gelbildners hängt von dem jeweils verwendeten Gelbildner ab. Werden Metallseifen als Gelbildner eingesetzt, so beträgt deren gewichtsprozentualer Anteil zwischen 0,5 und 25 Gew.% und vorzugsweise zwischen 2,5 und 20 Gew.% und besonders bevorzugt zwischen 4 und 8 Gew.% Da Metallseifen schlecht wasserlöslich sind und sich bei dem wenigstens zweiphasigen Mittel beide Phasen gleichzeitig auflösen sollen, ist man bestrebt, den Anteil der Metallseifen in der Gelphase in der Regel auf 4 bis 8 Gew.% einzustellen.

Bei Einsatz von Polyalkoxyalkanen als Gelbildner beträgt deren Anteil zwischen 10 und 40, vorzugsweise zwischen 15 und 35 Gew.%, beim Einsatz von natürlichen oder synthetischen "gums" werden vorzugsweise zwischen 0,1 und 10 Gew.% eingesetzt.

Ein weiterer wesentlicher Bestandteil der Gelphase ist das Lösungsmittel. Prinzipiell kann ein organisches Lösungsmittel, Wasser oder deren Gemisch als Lösungsmittel eingesetzt werden. Neben Wasser sind insbesondere Alkylenglycolalkylether wie beispielsweise Propylenglycol-n-Buthylether, Dipropylenglycol-Methylether oder deren Gemisch als Lösungsmittel geeignet. Auch können als Lösungsmittel C₃ - C₅ Glykole, wie insbesondere Propylenglykol (zum Beispiel 1,2-Propandiol), Propylencarbonate, C₂ - C₄ Alkohole oder Polyalkylenglycole, vorzugsweise mit 200 - 600 Alkylenglycoleinheiten, Polyalkohole, (2)-Methyl-1,3-Propandiol, auch in Mischungen anderer Lösungsmittel, verwendet werden. Ein weiteres bevorzugtes Lösungsmittel sind die als Emulgator bekannten Fettalkoholethoxylate und deren Derivate wie zum Beispiel Hydroxyfettalkoholalkoxylat, wobei der Alkoxylatrest ein Copolymer aus Ethylen- und Propylenoxideinheiten ist und der Alkylrest aus einem C12/C16-Blend, vorzugsweise deren 2-Hydroxyverbindung, besteht. Diese Gruppe der bevorzugten Lösungsmittel sind bei der Firma Cognis unter der Marke "Eumulgin", zum Beispiel Eumulgin L, erhältlich.

Das beziehungsweise die Lösungsmittel sind im Allgemeinen polare Lösungsmittel, die die Gelbildner lösen. Ihr prozentualer Anteil beträgt zwischen 5 und 70 Gew.%.

Ist ein Teil des Lösungsmittels gleichzeitig ein Emulgator, so kann der Lösungmittelanteil auf über 70 Gew.% steigen, zum Beispiel bis auf 80 Gew.%.

Der Gesamtgehalt an Lösungsmitteln in der Gelphase wird durch die für die Gelbildung mit dem jeweiligen Gelbildner erforderliche Lösungsmittelmenge bestimmt. Der Gesamtlösungsmittelgehalt der Gelphase schließt das in anderen Bestandteilen enthaltende Wasser, beispielsweise den Wassergehalt des zugesetzten Schäumers, mit ein. Im allgemeinen enthält die Gelphase in Abhängigkeit von dem eingesetzten Gelbildner wenigstens 25 Gew.% Lösungsmittel, vorzugsweise wenigstens 30 Gew.% und besonders bevorzugt mehr als 40 Gew.% Lösungsmittel.

Wird als Gelbildner eine Metallseife eingesetzt, so beträgt der Gesamtlösungsmittelanteil wenigstens 40 Gew.%, vorzugsweise mehr als 55 Gew.%. Als Lösungsmittel kann beispielsweise 0 bis 50 Gew.% Wasser zusammen mit 0 bis 60 Gew.%, beispielsweise 20 bis 25 Gew.% Wasser und 40 bis 50 Gew.% organisches Lösungsmittel, vorzugsweise aus der Gruppe der Alkylenglycolalkylether oder deren Mischung, eingesetzt werden. Auch ist möglich, als Lösungsmittelgemisch 0 bis 10 Gew.% Wasser, insbesondere 5 bis 8 Gew.% Wasser, sowie 10 bis 20 Gew.% C₃ bis C₅ Glycole, insbesondere 11 bis 14 Gew.% Propandiol und 45 bis 60 Gew.% Fettalkoholalkoxylat, insbesondere 50 bis 55 Gew.% Eumulgin L, einzusetzen.

Weiterhin enthält die Gelphase die zur permanenten Raumbeduftung erforderlichen Duftstoffe, insbesondere Parfümöle oder feste Riechstoffe wie Campher. Die eingesetzten Parfümöle sind vorzugsweise hydrophob. Der Parfümanteil in der Gelphase hängt von der gewünschten Beduftung ab. Im Allgemeinen beträgt der Parfümanteil zwischen 2 und 70 Gew.%, vorzugsweise zwischen 5 und 20 Gew.% und besonders bevorzugt 8 bis 12 Gew.%.

Für eine hocheffiziente Raumbeduftung wird der Parfumanteil der Gelphase auf 20 bis 30 Gew.% erhöht. Ein bevorzugtes Beduftungsmittel ist "Mint Fresh" der Firma Quest.

Unter Parfum werden sämtliche duftende Rohstoffe des Mittels verstanden, auch wenn sie gleichzeitig Lösungsmittel, Lösungsretardierer etc. sind.

Eine bevorzugte Ausführungsform sieht vor, dass die Gelphase zusätzlich einen oder mehrere Schäumer wie beispielsweise Betaine, alkoxylierte Alkylethersulfate oder Lactobionsäurederivate umfasst, wie beispielsweise Fettsäureamidopropyl-Betain mit einem C5 - C21-Fettsäureanteil wie beispielsweise Kokosamidoproplybetain, Alkali- oder Ammoniumsalze der Laurylethersulfate mit 1 bis 5 EO, Lactobionococylamid, Lactobionooleylamid, Lactobionotalgamid etc. oder deren Mischungen. Diese Schäumer lassen sich außerordentlich gut in die Gelphase einbringen. Besonders bevorzugt ist der Einsatz der flüssigen Superschäumer, nämlich der Betaine und der Laurylethersulfate.

Durch Einsatz von Schäumern in der Gelphase kann somit ein gutes Schaumverhalten erreicht werden, wohingegen ein Einsatz dieser flüssigen Superschäumer in der Phase der Reinigungsmittelformkörper, die im Allgemeinen extrudiert oder gepresst wird, infolge der Klebrigkeit der Schäumer schwer möglich ist.

Sofern zusätzlich in der Gelphase ein Schäumer eingesetzt wird, beträgt dessen Anteil vorzugsweise bis zu 10 Gew.%, vorzugsweise 3 bis 7 Gew.%.

Falls gewünscht, kann die Gelphase weiterhin Di-, Oligo- oder Polyhydroxyverbindungen oder deren Ether wie Glykol, 1,2- oder 1,3-Dihydroxypropan, 1,2-, 1,3-, 2,3- oder 1,4-Dihydroxybutan, die Isomere des Dihydroxyisobutans, Dihydroxypentans etc., Glycerin, Pentaerythrit, Penta- oder Hexahydroxyverbindungen oder Polyhydroxyether wie Polymethylen- oder Polypropylenglykol in einem Anteil von bis zu 20 Gew.% umfassen, um die Bildung schwerlöslicher Rückstände durch Austrocknung des Gels zu verhindern, den Auflösevorgang zu beschleunigen und eine ansehnlich glatte Oberfläche der Gelphase zu erreichen.

Falls gewünscht, kann die Gelphase zusätzlich Konservierungsmittel, z.B. Hydroxybenzoesäurealkylester wie Hydroxybenzoesäuremethyl- oder - propylester, umfassen. Im Allgemeinen beträgt deren Anteil zwischen 0 und 1 Gew.%.

Zur Steuerung der Auflösegeschwindigkeit können der Gelphase zusätzlich Hydrophobiermittel wie beispielsweise Kokosfettsäuremonoethanolamid oder andere bekannte Hydrophobiermittel zugegeben werden; bevorzugt sind, 0 bis 10 Gew.% Hydrophobiermittel einzusetzen. Prinzipiell tragen auch die in der Gelphase enthaltenen hydrophoben Parfümöle zu einer Verminderung der Auflösegeschwindigkeit bei.

Soll die Auflösegeschwindigkeit hingegen erhöht werden, so setzt man Hydrotropika ein. Geeignete Hydrotropika sind Natriumtoluolsulfonat, Natriumcumolsulfonat, Natriumxylolsulfonat oder auch Natrium-Butylmonoglykolsulfat. Anstelle der Natriumsalze können auch andere geeignete Salze, insbesondere Alkalisalze, eingesetzt werden.

Der gewichtsprozentuale Anteil der Hydrotropika hängt von der gewünschten Absenkung der Lebensdauer der Gelphase ab. Häufig liegt er zwischen 0 und 10 Gew.%.

Weiterhin kann die Gelphase weitere übliche Bestandteile wie Farbstoffe, Tenside, insbesondere schäumende Tenside, die kein all zu hohes Netzvermögen aufweisen, so dass sie sich nicht an die freizusetzenden Duftstoffe binden, Desinfektionsmittel oder auch Salze zur Steuerung der Auflösegeschwindigkeit umfassen.

Bevorzugt ist die Gelphase transparent.

Vorzugsweise sollte der Schmelzpunkt der Gelphase - um eine formstabile Lagerung und Transport des Mittels zu gewährleisten - wenigstens 40° C, bevorzugt wenigstens 50° C, betragen. Durch diese Mindestschmelzpunkte können bei der Herstellung erforderliche Kühlzeiten der zweiten Phase vor Eingießen der Gelphase reduziert werden.

### Zweite Phase (Reinigungsmittelformkörper)

Die zweite Phase ist die Phase des Reinigungsmittelformkörpers. Unter Reinigungsmittelformkörper wird ein fester Körper, der vorzugsweise durch Extrusion oder Verpressen entsprechend der üblichen Herstellung von Rimblocks hergestellt ist, verstanden.

Der Reinigungsmittelformkörper umfasst Tenside, um die gewünschte Reinigungswirkung zu erzielen. Diese Tenside sollten zur Erzielung der gewünschten Reinigung gut netzende Tenside, somit vorzugsweise anionische oder nichtionische Tenside, sein. Prinzipiell sind alle bekannten anionischen Tenside geeignet. Vorzugsweise werden als anionische Tenside Alkylbenzolsulfonate, Alkylsulfate, Fettalkoholsulfate und Fettalkoholethersulfate eingesetzt. Vorzugsweise umfasst die Alkylgruppe oder der Fettsäurebestandteil zwischen 8 und 18 Kohlenstoffatome. Als Alkylbenzolsulfonat kann beispielsweise Natrium-alkyl-(C10-C13)-benzolsulfonat eingesetzt werden.

Weiterhin kann die zweite Phase Salze zur Regulierung der Konsistenz und des Abspülverhaltens enthalten. Im Allgemeinen handelt es sich bei diesen Salzen um anorganische Salze, die vorzugsweise aus der Gruppe der Alkali- und Erdalkalisalze der Schwefelsäuren, Phosphorsäuren, Stickstoffsäuren, Kohlensäure, Halogensäuren wie HCl oder deren Mischungen ausgewählt werden. Der Anteil an diesen Salzen in der zweiten Phase kann bis zu 80 Gew.% betragen, vorzugsweise etwa 20 bis 50 Gew.%. Besonders bevorzugt ist, als Salze Natriumchlorid oder Natriumsulfat oder deren Mischung einzusetzen.

Die zweite Phase kann weiterhin Extrusionshilfsmittel, vorzugsweise bis zu einem Anteil von 15 Gew.%, umfassen. Als Extrusionshilfsmittel können beispielsweise Alkylpolyethylenglycolether mit bis zu 40 EO, aber auch andere bekannte Extrusionshilfsmittel wie zum Beispiel Cellulose und ihre Derivate verwendet werden.

Weiterhin kann die zweite Phase Farbstoffe und Pigment wie beispielsweise Titandioxid umfassen.

Wahlweise kann die zweite Phase auch amphotere Tenside enthalten.

Falls gewünscht, kann auch die zweite Phase Duftstoffe umfassen. Eine solche Ausführungsform ist bevorzugt, wenn ein Mittel bereitgestellt werden soll, das in einer über einen langen Zeitraum nicht benutzten Toilette eingesetzt werden soll. Durch die Gelphase werden die Duftstoffe permanent freigesetzt. Ist der Duftstoff der Gelphase nach einer längeren Zeit verbraucht und enthält auch die zweite Phase Duftstoffe, so werden bei Betätigung der Toilettenspülung die in der zweiten Phase befindlichen Duftstoffe abgespült und die gewünschte Beduftung erzielt. Durch einen gewissen Duftstoffanteil in der zweiten Phase kann somit für den Fall des Verbrauchs der Duftstoffe in der ersten Phase noch eine Grundbeduftung erreicht werden.

In einer anderen bevorzugten Ausführungsform ist die zweite Phase duftstofffrei. Somit lassen sich mit dieser universellen Phase unterschiedliche Produkte - nur abhängig von der Duftrichtung der Gelphase - herstellen.

Weiterhin kann die zweite Phase optional auch Desinfektionsmittel, Konservierungsmittel, Bleichmittel, Aktivatoren, Enzyme, Komplexierungsmittel und Säuren umfassen.

Neben diesen beiden Phasen kann das Mittel wahlweise noch weitere Phasen umfassen, beispielsweise eine dritte, der zweiten nicht gelförmigen Phase ähnliche Phase einer anderen Farbe mit Tensiden oder eine weitere, gegebenenfalls anders eingefärbte Gelphase.

Das Mittel, das die wenigstens zwei Phasen aufweist, ist üblicherweise so geformt, dass es in ein herkömmliches, am Rand der Toilette befestigbares Toilettenkörbchen eingebracht werden kann. Vorzugsweise weist das Mittel eine annähernd zylindrische oder quaderförmige Form auf.

Eine bevorzugte Ausführungsform sieht vor, die zweite Phase, nämlich den Reinigungsmittelformkörper, im wesentlichen quaderförmig auszubilden, wobei mindestens eine Seite des Quaders einen Freiraum beziehungsweise eine Vertiefung zur Aufnahme der Gelphase aufweist. Unter im wesentlichen quaderförmig wird auch ein "Quader" verstanden, dessen eine oder mehrere Kanten abgerundet oder mit einem Radius versehen sind. Der Freiraum bzw. die Vertiefung können beispielsweise konkav oder zylindrisch geformt sein. Auch ist es möglich, den Freiraum beispielsweise figürlich auszubilden. Prinzipiell ist es ebenfalls möglich, dass das Mittel zylindrisch oder auch quaderförmig ist und beidseitig einer Ebene der Form des Mittel die erste und die zweite Phase aufweist.

Zur Herstellung des erfindungsgemäßen Mittels werden zunächst die Bestandteile der zweiten Reinigungsmittelformköperphase gemischt, anschließend extrudiert und in zylindrische oder quaderförmige Stücke geschnitten. Dann wird auf wenigstens einer Seite des Formkörpers die Vertiefung beziehungsweise der Freiraum erzeugt, beispielsweise durch einen Pressvorgang, und anschließend das aus den Komponenten der Gelphase hergestellte aufgeschmolzene Gel in den Freiraum beziehungsweise die Vertiefung eingefüllt.

Durch geeignete Prägung oder Formung der Vertiefung beziehungsweise des Freiraums kann zusätzlich eine weitere Individualisierung des Mittels gewährleistet werden, beispielsweise durch Prägung der Vertiefung in Form des Unternehmenskennzeichens oder der Marke.

Weiterhin ist es denkbar, in die Vertiefung beziehungsweise den Freiraum des Reinigungsmittelformkörpers einen sogenannten "End-of-life"-Indikator einzulegen, der dann mit der Gelphase übergossen wird. Bei diesem "End-of-life"-Indikator kann es sich beispielsweise um ein Kunststoffplättchen handeln, das - wenn es nach Verbrauch des Mittels freigespült ist - durch entsprechende Optik dem Verbraucher im oder außerhalb des Körbchens den Verbrauch visualisiert.

Ein weiteres Ausführungsbeispiel sieht vor, als "End-of-life"-Indikator einen hochparfümierten Pressling einzulegen, der - sofern er freigespült ist - durch eine kurz anhaltende starke Parfümierung den Konsumenten an ein Nachfüllen erinnert.

Ebenfalls ist es möglich, beispielsweise mittels elektrischer Bauteile ein akustisches "End-of-life"-Signal bereitzustellen.

Eine weitere bevorzugte Ausführungsform sieht vor, den Reinigungsmittelformkörper in Form eines Rohres zu extrudieren, die innere Öffnung des Reinigungsmittelformkörperextrudates anschließend mit der Gelphase zu befüllen und das gelbefüllte Rohr dann in einzelne Stück zu zerschneiden, die in die Toilettenkörbchen befestigbar sind.

Das Rohr kann verschiedenartig gestaltet sein, zum Beispiel zylindrisch oder außen und innen eckig oder außen eckig und innen gerundet etc.

Um ein Herausfallen der Gelkomponente in der Toilette, insbesondere während des Spülens, zu vermeiden, weist die Innenfläche des Reinigungsmittelformkörpers vorzugsweise eine geeignete, lokal unsymmetrische Form, die sich vorzugsweise schraubenförmig entlang der Innenfläche erstreckt, auf, durch die sich die Gelphase gegenüber dem Reinigungsmittelformkörper verkeilt. Ein solches Verkeilen der beiden Phasen kann beispielsweise dadurch erreicht werden, dass der Dorn der Extrusionsdüse eine Nase aufweist und die zu extrudierende Masse mit einem Drall auf die Extrusionsdüse auftrifft.

Die Erfindung wird nachfolgend anhand zweier Rezepturen näher beschrieben.

### 1. Ausführungsbeispiel

### a) Für die Gelphase wurden folgende Rohstoffe eingesetzt:

| **Rohstoff** | | **Funktion** | **Gew.%** |
|---|---|---|---|
| Dowanol PnB der Fa. Dow Chemicals | Propylenglycol-n-Buthylether | Lösungsmittel | 11 |
| Dowanol DPM der Fa. Dow Chemicals | Dipropylenglycol-Methylether | Lösungsmittel | 34 |
| Natriumstearat der Fa. Bärlocher | Natriumstearat | Gelbildner | 8 |
| Tego Betain F50 der Fa. Goldschmidt | Kokosamidopropylbetain (44% in Wasser) | Schäumer | 5 |
| Comperlan 100 der Fa. Cognis | Kokosfettsäuremonoethanolamid | Hydrophobierer | 5 |
| Texapon N70 der Fa. Cognis | Natriumlaurylethersulfat (2 EO) 70 % in Wasser | Schäumer | 19 |
| Solbrol M der Fa. Bayer | p-Hydroxybenzoesäuremethylester | Konservierungsmittel | 0,08 |
| Solbrol P der Fa. Bayer | p-Hydroxybenzoesäurepropylester | Konservierungsmittel | 0,12 |
| Parfüm | Parfüm | Duftstoff | 10 |
| Wasser | | | ad 100 |

### b) Rahmenrezeptur für die zweite Phase (Reinigungsmittelformkörper)

| **Rohstoff** | **Funktion** | **Bereich Gew.%** | **z. B. Gew.%** |
|---|---|---|---|
| sec. Alkyl-(C10-13)-benzol-sulfonat-Natriumsalz | Anionisches Tensid | 15- 25 | 20 |
| Natriumchlorid | Salz | 15-35 | 35 |
| Natriumsulfat | Salz | 15- 25 | ad 100 |
| Alkyl-(C13,15)-polyethylen-glycolether (5 EO) | Nichtionisches Tensid, Extrusionshilfsmittel | 2 - 7 | 2 |
| Alkyl-(C16-18)-polyethylen-glycolether (25 EO) | Nichtionisches Tensid, Extrusionshilfsmittel | 1-6 | 6 |
| Farbstoff | Farbstoff | 0 - 1 | 0,005 |
| Titandioxid | Farbstoffpigment | 0 - 1 | 0,5 |
| Parfüm | Parfüm | 0-8, insb. 3-8 | 2 |

### 2. Ausführungsbeispiel eines stark duftenden Mittels

### a) Gelphase

| | Rohstoff | chemische Verbindung | Zweck | Menge [%] |
|---|---|---|---|---|
| 1. | Wasser | Wasser | Lösungsmittel | 5,0 |
| 2. | Propylenglykol techn. | 1,2-Propandiol | Lösungsmittel | 12,5 |
| 3. | Eumulgin L | Hydroxyfettalkoholalkoxylat (1) | Emulgator/ Lösungsmittel | 53,0 |
| 4. | Na-Stearat | Na-Stearat | Verdicker | 5,0 |
| 5. | Hydriosul BN 100 | Na-Butylmonoglykolsulfat, | Abspülregulator | 4,5 |
| 6. | Duftstoff Mint Fresh | Duftstoffkomposition | Beduftung | 20 |

| | | | | |
|---|---|---|---|---|
| (1) Alkyl (C12/C16)-2-hydroxy + 9 EO + 1,2 PO | | | | |

### b) Reinigungsmittelformkörper

| **Rohstoff** | **Funktion** | |
|---|---|---|
| sec. Alkyl-(C10-13)-benzol-sulfonat-Natriumsalz | Anionisches Tensid | 25 |
| Natriumchlorid | Salz | 35 |
| Natriumsulfat | Salz | ad 100 |
| Alkyl-(C13,15)-polyethylen-glycolether (5 EO) | Nichtionisches Tensid, Extrusionshilfsmittel | 2 |
| Alkyl-(C16-18)-polyethylen-glycolether (25 EO) | Nichtionisches Tensid, Extrusionshilfsmittel | 6 |
| Farbstoff | Farbstoff | 0,005 |
| Titandioxid | Farbstoffpigment | 0,5 |
| Parfüm | Parfüm | 0 |

## Patentansprüche

1. Toilettenreinigungs- und Beduftungsmittel, welches Mittel wenigstens zwei Phasen umfasst, wobei die erste Phase eine Gelphase ist, die Gelbildner, Duftstoffe und Lösungsmittel umfasst und die zweite Phase ein nicht gelförmiger Reinigungsmittelformkörper ist, der Tenside umfasst.

2. Mittel nach Anspruch 1, **dadurch gekennzeichnet, dass** die Gelphase ein definiertes Abdampfprofil aufweist.

3. Mittel nach Anspruch 1, **dadurch gekennzeichnet, dass** der Gelbildner aus der Gruppe der Metallseifen der höheren Fettsäuren, insbesondere deren Alkalisalze wie Natriumstearat oder Natriumoleat, der nichtionischen Tenside, insbesondere der Polyalkoxyalkane, der natürlichen Polymere ("gums"), insbesondere Gummi arabicum, Guar-Gum, Agar Agar, Pectine, Gelatine, Stärke, Sorbitol, der chemisch modifizierten oder derivatisierten natürliche gums wie Carboxymethylcellulose, Methylcellulose, Hydroxypropylmethylcellulose, Xanthan, der Sorbitolderivate, der Stärkederivate wie Carboxymethylstärke, Hydroxyethylstärke, der mikrobiell fermentierten "gums" wie Dextran, Polysaccharid B-1459 sowie Methoxypectin, Propylenglycolalginat, Triethanolaminalginat und Carboxymethyl-Guar-Gum ausgewählt wird.

4. Mittel nach Anspruch 3, **dadurch gekennzeichnet, dass** der gewichtsprozentuale Anteil der Metallseifen als Gelbildner zwischen 0,5 und 25 Gew.% und vorzugsweise zwischen 2,5 und 20 Gew.% beträgt.

5. Mittel nach Anspruch 3, **dadurch gekennzeichnet, dass** bei Einsatz von Polyalkoxyalkanen als Gelbildner deren Anteil zwischen 10 und 40, vorzugsweise zwischen 15 und 35 Gew.% und beim Einsatz von natürlichen oder synthetischen gums deren Anteil zwischen 0,1 und 10 Gew.% beträgt.

6. Mittel nach einem der Ansprüche 1 bis 5, **dadurch gekennzeichnet, dass** das Lösungsmittel der Gelphase ein organisches Lösungsmittel, Wasser oder deren Gemisch ist, wobei das organische Lösungsmittel insbesondere aus der Gruppe der Alkylenglycolalkylether, der C₃ - C₅ Glykole, Propylencarbonate, C₂ - C₄ Alkohole, Polyalkylenglycole, Polyalkoholen und 2-Methyl-1,3-Propandiol ausgewählt wird.

7. Mittel nach Anspruch 6, **dadurch gekennzeichnet, dass** der prozentuale Anteil des beziehungsweise der Lösungsmittel zwischen 5 und 80 Gew.%, insbesondere bis zu 70 Gew.%, insbesondere wenigstens 25 Gew.%, vorzugsweise wenigstens 30 Gew.% und besonders bevorzugt mehr als 40 Gew.%, beträgt.

8. Mittel nach einem der Ansprüche 1 bis 7, **dadurch gekennzeichnet, dass** der Parfümanteil der Gelphase zwischen 2 und 70 Gew.%, vorzugsweise zwischen 5 und 20 Gew.% und besonders bevorzugt 8 bis 12 Gew.% beträgt.

9. Mittel nach einem der Ansprüche 1 bis 8, **dadurch gekennzeichnet, dass** die Gelphase zusätzlich einen oder mehrere Schäumer, insbesondere Betaine, alkoxylierte Alylethersulfate oder Lactobionsäurederivate, und/oderDi-, Oligo- oder Polyhydroxyverbindungen oder deren Ether, insbesondere Glykol, 1,2- oder 1,3-Dihydroxypropan, 1,2-, 1,3-, 2,3- oder 1,4-Dihydroxybutan, die Isomere des Dihydroxyisobutans, Dihydroxypentans etc., Glycerin, Pentaerythrit, Penta- oder Hexahydroxyverbindungen oder Polyhydroxyether wie Polymethylen- oder Polypropylenglykol, insbesondere in einem Anteil von bis zu 20 Gew.%, umfasst.

10. Mittel nach einem der Ansprüche 1 bis 9, **dadurch gekennzeichnet, dass** der Schmelzpunkt der Gelphase wenigstens 40°C, vorzugsweise wenigstens 50°C, beträgt.

11. Mittel nach einem der Ansprüche 1 bis 10, **dadurch gekennzeichnet, dass** die Tenside der zweiten Phase anionische und/oder nichtionische Tenside, vorzugsweise Alkylbenzolsulfonate, Alkylsulfate, Fettalkoholsulfate und/oder Fettalkoholethersulfate, sind.

12. Mittel nach einem der Ansprüche 1 bis 11, **dadurch gekennzeichnet, dass** die zweite Phase Salze, insbesondere anorganische, vorzugsweise aus der Gruppe der Alkali- und Erdalkalisalze der Schwefelsäuren, Phosphorsäuren, Stickstoffsäuren, Kohlensäure, Halogensäuren wie HCl oder deren Mischungen, umfasst und der Anteil der Salze insbesondere bis zu 80 Gew.%, vorzugsweise etwa 20 bis 50 Gew.%, beträgt.

13. Mittel nach einem der Ansprüche 1 bis 12, **dadurch gekennzeichnet, dass** die zweite Phase im wesentlichen quaderförmig oder zylindrisch ausgebildet ist, wobei mindestens eine Seite des Quaders oder Zylinders einen Freiraum beziehungsweise eine Vertiefung zur Aufnahme der Gelphase aufweist, wobei die Vertiefung beziehungsweise der Freiraum insbesondere einen "End-of-life"-Indikator umfasst.

14. Verfahren zur Herstellung eines Mittels nach einem der Ansprüche 1 bis 13, **dadurch gekennzeichnet, dass** die Bestandteile der zweiten Reinigungsmittelformköperphase gemischt, anschließend extrudiert und in zylindrische oder quaderförmige Stücke geschnitten, dann auf wenigstens einer Seite des Formkörpers die Vertiefung beziehungsweise der Freiraum erzeugt und anschließend das aus den Komponenten der Gelphase hergestellte aufgeschmolzene Gel in den Freiraum beziehungsweise die Vertiefung eingefüllt wird.

15. Verfahren zur Herstellung eines Mittels nach einem der Ansprüche 1 bis 13, **dadurch gekennzeichnet, dass** der Reinigungsmittelformkörper in Form eines Rohres extrudiert wird, die innere Öffnung des Reinigungsmittelformkörperextrudats anschließend mit einer Gelphase befüllt und das gelgefüllte Rohr dann in einzelne Stücke zerschnitten wird.

## Claims

1. Toilet cleaning and odorising agent, which agent comprises at least two phases where the first phase is a gel phase which comprises gel formers, fragrances and solvents and the second phase is a non-gel cleaning agent shaped-body which comprises surfactants.

2. Agent according to Claim 1, **characterized in that** the gel phase has a defined evaporation profile.

3. Agent according to Claim 1, **characterized in that** the gel former is chosen from the group of metal soaps of higher fatty acids, in particular their alkali metal salts, such as sodium stearate or sodium oleate, nonionic surfactants, in particular polyalkoxyalkanes, natural polymers ("gums"), in particular gum arabic, guar gum, agar agar, pectins, gelatin, starch, sorbitol, chemically modified or derivatized natural gums, such as carboxymethylcellulose, methylcellulose, hydroxypropylmethylcellulose, xanthan, sorbitol derivatives, starch derivatives, such as carboxymethyl starch, hydroxyethyl starch, microbially fermented "gums", such as dextran, polysaccharide B-1459, and methoxypectin, propylene glycol alginate, triethanolamine alginate and carboxymethyl guar gum.

4. Agent according to Claim 3, **characterized in that** the weight percentage fraction of the metal soaps as gel formers is between 0.5 and 25% by weight and preferably between 2.5 and 20% by weight.

5. Agent according to Claim 3, **characterized in that** when using polyalkoxyalkanes as gel formers their fraction is between 10 and 40% by weight, preferably between 15 and 35% by weight, and when using natural or synthetic gums their fraction is between 0.1 and 10% by weight.

6. Agent according to one of Claims 1 to 5, **characterized in that** the solvent of the gel phase is an organic solvent, water or mixture thereof, where the organic solvent is chosen in particular from the group of alkylene glycol alkyl ethers, C₃-C₅ glycols, propylene carbonates, C₂-C₄ alcohols, polyalkylene glycols, polyalcohols and 2-methyl-1,3-propanediol.

7. Agent according to Claim 6, **characterized in that** the percentage fraction of the solvent or solvents is between 5 and 80% by weight, in particular up to 70% by weight, in particular at least 25% by weight, preferably at least 30% by weight and particularly preferably more than 40% by weight.

8. Agent according to one of Claims 1 to 7, **characterized in that** the perfume fraction of the gel phase is between 2 and 70% by weight, preferably between 5 and 20% by weight and particularly preferably 8 to 12% by weight.

9. Agent according to one of Claims 1 to 8, **characterized in that** the gel phase additionally comprises one or more foamers, in particular betaines, alkoxylated alkyl ether sulphates or lactobionic acid derivatives, and/or di-, oligo- or polyhydroxy compounds or ethers thereof, in particular glycol, 1,2- or 1,3-dihydroxypropane, 1,2-, 1,3-, 2,3- or 1,4-dihydroxybutane, the isomers of dihydroxyisobutane, dihydroxypentane etc., glycerol, pentaerythritol, penta- or hexahydroxy compounds or polyhydroxy ethers, such as polymethylene glycol or polypropylene glycol, in particular in a fraction of up to 20% by weight.

10. Agent according to one of Claims 1 to 9, **characterized in that** the melting point of the gel phase is at least 40°C, preferably at least 50°C.

11. Agent according to one of Claims 1 to 10, **characterized in that** the surfactants of the second phase are anionic and/or nonionic surfactants, preferably alkylbenzenesulphonates, alkyl sulphates, fatty alcohol sulphates and/or fatty alcohol ether sulphates.

12. Agent according to one of Claims 1 to 11, **characterized in that** the second phase comprises salts, in particular inorganic ones, preferably from the group of alkali metal and alkaline earth metal salts of sulphuric acids, phosphoric acids, nitrogen acids, carbonic acid, halogen acids, such as HCl or mixtures thereof, and the fraction of salts is in particular up to 80% by weight, preferably about 20 to 50% by weight.

13. Agent according to one of Claims 1 to 12, **characterized in that** the second phase is essentially cuboidal or cylindrical in shape, where at least one side of the cuboid or cylinder has a cavity or a hollow for accommodating the gel phase, where the hollow or the cavity comprises in particular an "end-of-life" indicator.

14. Process for the preparation of an agent according to one of Claims 1 to 13, **characterized in that** the constituents of the second cleaning agent shaped-body phase are mixed, then extruded and cut into cylindrical or cuboidal pieces, then on at least one side of the shaped-body the hollow or the cavity is produced and then the molten gel prepared from the components of the gel phase is poured into the cavity or the hollow.

15. Process for the preparation of an agent according to one of Claims 1 to 13, **characterized in that** the cleaning agent shaped-body is extruded in the form of a tube, the inner opening of the cleaning agent shaped-body extrudate is then filled with a gel phase and the gel-filled tube is then cut into individual pieces.

## Revendications

1. Composition de nettoyage et d'apport de parfum, laquelle composition comprend au moins deux phases, la première phase étant une phase de type gel qui comprend des agents gélifiants, des parfums et des solvants, et la seconde phase étant un corps moulé de produit de nettoyage non sous forme de gel, qui comprend des tensioactifs.

2. Composition selon la revendication 1, **caractérisée en ce que** la phase de type gel présente un profil d'évaporation défini.

3. Composition selon la revendication 1, **caractérisée en ce que** l'agent gélifiant est choisi dans le groupe constitué par les savons métalliques des acides gras supérieurs, en particulier leurs sels alcalins, tels que le stéarate de sodium ou l'oléate de sodium, les tensioactifs non ioniques, en particulier les polyalcoxyalcanes, des polymères naturels ("gommes"), en particulier la gomme arabique, la gomme guar, l'agar-agar, les pectines, la gélatine, l'amidon, le sorbitol, les gommes naturelles modifiées ou fonctionnalisées chimiquement, telles que la carboxyméthylcellulose, la méthylcellulose, l'hydroxypropylméthylcellulose, le xanthane, les dérivés de sorbitol, les dérivés d'amidon tels que le carboxyméthylamidon, l'hydroxyéthylamidon, des "gommes" fermentées microbiologiquement, telles que le dextrane, le polysaccharide B-1459 ainsi que la méthoxypectine, le propylèneglycolalginate, le triéthanolamine-alginate et la gomme guar carboxyméthylée.

4. Composition selon la revendication 3, **caractérisée en ce que** la proportion en % en poids des savons métalliques en tant qu'agent gélifiant est comprise entre 0,5 et 25 % en poids et de préférence entre 2,5 et 20 % en poids.

5. Composition selon la revendication 3, **caractérisée en ce que** dans le cas de l'utilisation de polyalcoxyalcanes en tant qu'agent gélifiant, leur proportion est comprise entre 10 et 40, de préférence entre 15 et 35 % en poids, et dans le cas de l'utilisation de gommes naturelles ou synthétiques, leur proportion est comprise entre 0,1 et 10 % en poids.

6. Composition selon l'une quelconque des revendications 1 à 5, **caractérisée en ce que** le solvant de la phase de type gel est un solvant organique, l'eau ou un mélange de ceux-ci, le solvant organique étant choisi en particulier dans le groupe constitué par les alkylèneglycolalkyléthers, les glycols en C₃-C₅, les propylènecarbonates, les alcools en C₂-C₄, les polyalkylèneglycols, les polyols et le 2-méthyl-1,3-propanediol.

7. Composition selon la revendication 6, **caractérisée en ce que** la proportion en pourcentage du ou des solvants est comprise entre 5 et 80 % en poids, en particulier va jusqu'à 70 % en poids, en particulier est d'au moins 25 % en poids, de préférence d'au moins 30 % en poids et de façon particulièrement préférée de plus de 40 % en poids.

8. Composition selon l'une quelconque des revendications 1 à 7, **caractérisée en ce que** la teneur en parfum de la phase de type gel est comprise entre 2 et 70 % en poids, de préférence entre 5 et 20 % en poids et de façon particulièrement préférée va de 8 à 12 % en poids.

9. Composition selon l'une quelconque des revendications 1 à 8, **caractérisée en ce que** la phase de type gel comprend additionnellement un ou plusieurs agents moussants, en particulier des bétaïnes, des alkyléthersulfates alcoxylés ou des dérivés d'acide lactobioniques et/ou des composés di-, oligo- ou polyhydroxylés ou leurs éthers, en particulier le glycol, le 1,2-ou 1,3-dihydroxypropane, le 1,2-, 1,3-, 2,3- ou 1,4-dihydroxybutane, les isomères du dihydroxy-isobutane, du dihydroxypentane, etc., le glycérol, le pentaérythritol, des composés penta- ou hexahydroxylés ou des polyhydroxyéthers tels que le polyméthylène- ou polypropylèneglycol, en particulier en une proportion allant jusqu'à 20 % en poids.

10. Composition selon l'une quelconque des revendications 1 à 9, **caractérisée en ce que** le point de fusion de la phase de type gel est d'au moins 40°C, de préférence d'au moins 50°C.

11. Composition selon l'une quelconque des revendications 1 à 10, **caractérisée en ce que** les tensioactifs de la seconde phase sont des tensioactifs anioniques et/ou non ioniques, de préférence des alkylbenzènesulfonates, des alkylsulfates, des sulfates d'alcools gras et/ou des éthersulfates d'alcools gras.

12. Composition selon l'une quelconque des revendications 1 à 11, **caractérisée en ce que** la seconde phase comprend des sels, en particulier minéraux, de préférence choisis dans le groupe des sels alcalins et alcalino-terreux des acides soufrés, des acides phosphorés, des acides azotés, de l'acide carbonique, des acides halogènes tels que HCl ou des mélanges de ceux-ci, et la proportion des sels va en particulier jusqu'à 80 % en poids, de préférence d'environ 20 à 50 % en poids.

13. Composition selon l'une quelconque des revendications 1 à 12, **caractérisée en ce que** la seconde phase est essentiellement en forme d'un parallélépipède rectangle ou cylindrique, au moins une face du parallélépipède rectangle ou du cylindre présentant un espace libre ou un évidement pour la réception de la phase de type gel, l'évidement ou l'espace libre comprenant particulier un indicateur de fin d'utilisation ("end-of-life").

14. Procédé pour la production d'une composition selon l'une quelconque des revendications 1 à 13, **caractérisé en ce que** les composants de la seconde phase consistant en un corps moulé de produit de nettoyage sont mélangés, ensuite extrudés et le produit d'extrusion est découpé en tronçons cylindriques ou cubiques, puis l'évidement ou l'espace libre est formé sur au moins une face du corps moulé et l'espace libre ou, respectivement, l'évidement fondu, préparé à partir des composants de la phase de type gel, est ensuite rempli.

15. Procédé pour la production d'une composition selon l'une quelconque des revendications 1 à 13, **caractérisée en ce que** le corps moulé consistant en produit de nettoyage est extrudé sous forme d'un tube, l'espace vide interne de l'extrudat de corps moulé consistant en produit de nettoyage est ensuite rempli avec une phase de type gel, et le tube rempli de gel est ensuite découpé en tronçons individuels.
